# EUROPEAN PATENT SPECIFICATION

(11) **EP 3 553 789 B1**
(45) Date of publication and mention of the grant of the patent: **16.08.2023**
(21) Application number: 17879144.8
(22) Date of filing: 06.12.2017
(51) Int. Cl.: G16H 50/20, G16H 30/40

(54) **SYSTEM FOR DIAGNOSING DISEASE USING NEURAL NETWORK AND METHOD THEREFOR**
SYSTEM ZUR DIAGNOSE VON ERKRANKUNGEN MIT EINEM NEURONALEN NETZ UND VERFAHREN DAFÜR
SYSTÈME DE DIAGNOSTIC D'UNE MALADIE À L'AIDE D'UN RÉSEAU NEURONAL ET PROCÉDÉ ASSOCIÉ

(30) Priority: 11.12.2016 KR 20160168176
(43) Date of publication of application: 16.10.2019
(73) Proprietor: Deep Bio Inc., Seoul 08394 (KR)
(72) Inventor: KIM, Sun Woo, Gyeonggi-do 13599 (KR)
(74) Representative: Flügel Preissner Schober Seidel
(86) International application number: PCT/KR2017/014219
(87) International publication number: WO 2018/106005

(56) References cited:
- WO-A1-2015/177268
- WO-A1-2016/193979
- JP-A- 2008 536 211
- JP-A- 2011 112 523
- JP-A- 2014 049 118
- KR-A- 20160 034 814
- KR-B1- 101 563 569
- US-A- 6 004 267
- PETER LIU ET AL: "A prostate cancer computer-aided diagnosis system using multimodal magnetic resonance imaging and targeted biopsy labels", SPIE - INTERNATIONAL SOCIETY FOR OPTICAL ENGINEERING. PROCEEDINGS, vol. 8670, 26 February 2013 (2013-02-26), page 86701G, XP055699587, US ISSN: 0277-786X, DOI: 10.1117/12.2007927 ISBN: 978-1-5106-3377-3

## Description

### TECHNICAL FIELD

The present invention relates to a disease diagnosis system using a neural network and a method thereof, and more specifically, to a system capable of diagnosing a predetermined disease (e.g., prostate cancer), when an image of a biological tissue is inputted, using a well-trained neural network and a method thereof.

### BACKGROUND ART

One of the important tasks performed by pathology or pathology department is performing a diagnosis for determining a state or a symptom of a specific disease by reading a biometric image of a patient. Such a diagnosis is a method relying on the experience and knowledge of medical workers trained for an extended period of time.

Recently, attempts for automating a work such as recognizing or classifying an image using a computer system are actively made owning to development of machine learning. Particularly, attempts are made to automate diagnosis performed by skilled medical workers using a neural network (e.g., a deep learning method using a convolution neural network (CNN)), which is a kind of machine learning.

Particularly, since the diagnosis through deep learning using a neural network (e.g., CNN) is not simply automating the experience and knowledge of skilled medical workers in a conventional method, but finding out distinctive factors and deriving a desired answer through self-learning, there are occasions in which features of a disease factor unknown to the skilled medical workers are found in an image.

Generally, diagnosis of a disease through a neural network using a biomedical image uses a segment of the biomedical image, i.e., a tile. That is, a skilled medical worker annotates a state of a specific disease (e.g., whether a cancer has been manifested) to a corresponding tile and trains the neural network using a plurality of annotated tiles as training data. At this point, a convolution neural network may be used as the neural network.

However, in this case, the trained neural network determines a state of a disease of a corresponding tile using only the image features of the tile. However, in reality, when a state of a specific biological tissue is determined for a specific disease, there are many occasions in which states (e.g., a shape, whether a specific pattern exists and the like) of the tissues surrounding the specific biological tissue, as well as the specific biological tissue itself, should be considered together. However, the conventional method has a problem of not being relevant in this case.

WO 2016/193979 A1 discloses a brain computer interface and, more particularly, a method of classifying an image. The method comprises: applying a computer vision procedure to the image to detect therein candidate image regions suspected as being occupied by a target; presenting to an observer each candidate image region as a visual stimulus, while collecting neurophysiological signals from a brain of the observer; processing the neurophysiological signals to identify a neurophysiological event indicative of a detection of the target by the observer; and determining an existence of the target in the image is based, at least in part, on the identification of the neurophysiological event.

US 6 004 267 A discloses a method for diagnosing prostate cancer and determining preoperatively the pathological stage in patients with prostate cancer. The method can be used for prediction of margin positivity, seminal vesicle (S.V.) involvement, and lymph nodal (L.N.) involvement in patients with clinically localized prostate cancer. The method includes use of a neural network which provides prostate cancer stage information for a patient based upon input data which includes the patient's preoperative serum PSA, biopsy Gleason score, and systemic biopsy-based information. Its positive predictive value (PPV), negative predictive value (NPV), and accuracy are superior to that of current nomograms in use.

### DISCLOSURE OF INVENTION

### TECHNICAL PROBLEM

Therefore, the present invention has been made in view of the above problems, and it is an object of the present invention to provide a diagnosis system using a neural network and a method thereof, which can further enhance accuracy by using neighboring tiles, as well as a specific tile, for learning to determine the states (e.g., whether a disease has been manifested, an index indicating a state of a disease, and the like) of a disease of the specific tile.

Another object of the present invention is to provide a diagnosis system using a neural network and a method thereof, which can output information on a factor working as the basis for determining a state of a disease of a tile, not simply outputting information on the state.

### TECHNICAL SOLUTION

To accomplish the above objects, according to one aspect of the present invention, there is provided a disease diagnosis system implemented in a system including a processor and a storage device for storing a neural network and using a biometric image and the neural network, the disease diagnosis system including: a first, referred to as micro-neural network for receiving a predetermined tile included in the biometric image through an input layer and including a plurality of first layers and an output layer; and a second, referred to as macro-neural network for receiving a macro-tile including the tile and at least one of tiles adjacent to the tile through an input layer and including a plurality of second layers and the output layer, wherein the output layer includes at least a state channel indicating a state of a disease of a biological tissue corresponding to the tile.

The output layer is determined on the basis of output data of a first right-before layer included in the first layers and located right before the output layer and a second right-before layer included in the second layers and located right before the output layer.

The macro-neural network has a big stride compared with the micro-neural network.

The output layer may include: the state channel; and at least a correlation factor channel indicating a degree of manifestation of a correlation factor associated with a value of the state channel.

The disease may be prostate cancer.

In accordance with the invention, the state channel is a channel indicating a probability of a biomedical tissue corresponding to the tile to have a Gleason pattern value of a predetermined range.

The correlation factor channel may include at least a channel among a channel indicating a probability that a cell nucleus of a biological tissue corresponding to the tile satisfies a specific condition; a channel indicating a probability that a biological tissue corresponding to the tile is classified as a single cell layer; a channel indicating a probability that a biological tissue corresponding to the tile is classified as a high density gland; and a channel indicating a probability that a biological tissue corresponding to the tile is classified as normal stroma.

According to another aspect of the present invention, there is provided a disease diagnosis system implemented in a system including a processor and a storage device for storing a neural network and using a biometric image and the neural network, the disease diagnosis system including: an input layer for receiving a predetermined tile included in the biometric image; a plurality of layers; and an output layer, wherein the output layer includes at least a state channel indicating a state of a disease of a biological tissue corresponding to the tile, and at least a correlation factor channel indicating a degree of manifestation of a correlation factor associated with a value of the state channel.

According to another aspect of the present invention, there is provided a method of diagnosing a disease using a neural network, performed by a disease diagnosis system implemented in a system including a processor and a storage device and using a biometric image and the neural network, the method including the steps of: storing a micro-neural network for receiving a predetermined tile included in the biometric image through an input layer and including a plurality of first layers and an output layer, and a macro-neural network for receiving a macro-tile including the tile and at least one of tiles adjacent to the tile through an input layer and including a plurality of second layers and the output layer; and training the micro-neural network and the macro-neural network using annotation information annotated to the tile to correspond to the tile and the output layer, wherein the output layer includes at least a state channel indicating a state of a disease of a biological tissue corresponding to the tile.

The method of diagnosing a disease using a neural network may further include the steps of: receiving a target tile included in a diagnosis target biometric image, by the neural network including the trained micro-neural network and macro-neural network; and outputting an output data corresponding to the output layer through the neural network.

The output layer is determined on the basis of output data of a first right-before layer included in the first layers and located right before the output layer and a second right-before layer included in the second layers and located right before the output layer.

According to another aspect of the present invention, there is provided a method of diagnosing a disease using a neural network, performed by a disease diagnosis system implemented in a system including a processor and a storage device for storing the neural network and using a biometric image and the neural network, the method comprising the steps of: storing the neural network including an input layer for receiving a predetermined tile included in the biometric image, a plurality of layers, and an output layer; and training the neural network using the tile and annotation information of the tile corresponding the output layer, wherein the output layer includes at least a state channel indicating a state of a disease of a biological tissue corresponding to the tile, and at least a correlation factor channel indicating a degree of manifestation of a correlation factor associated with a value of the state channel.

The method may be implemented through a computer program installed in a data processing device and hardware of the data processing device which can execute the computer program.

### ADVANTAGEOUS EFFECTS

According to the present invention, there is an effect of providing further higher accuracy of diagnosis by providing a neural network which can determine a state of a disease of a specific tile considering also a macro-tile including the specific tile and further including neighboring tiles while performing the diagnosis on the specific tile.

In addition, there is an effect of enhancing reliability of information on a disease determined by a diagnosis system since a neural network does not simply output only information on the state of the disease, but further outputs information on the factors associated with the state, and there is an effect of verifying incorrect determination by grasping information on the state and the ground thereof.

### BRIEF DESCRIPTION OF THE DRAWINGS

To further sufficiently understand the drawings adopted in the detailed description of the present invention, the drawings are briefly described.
FIG. 1 is a view showing the schematic system configuration of a disease diagnosis system using a neural network according to the present invention.
FIG. 2 is a view showing the logical configuration of a disease diagnosis system using a neural network according to an embodiment of the present invention.
FIG. 3 is a view showing the hardware configuration of a disease diagnosis system using a neural network according to an embodiment of the present invention.
FIG. 4 is a view showing the configuration of a neural network according to an embodiment of the present invention.
FIG. 5 is a view showing an output layer of a neural network according to an embodiment of the present invention.
FIG. 6 is a view showing an example of annotation information for training a neural network according to an embodiment of the present invention.
FIGS. 7 to 10 are views showing correlation factors in a neural network according to an embodiment of the present invention.
FIG. 11 is a view showing a result of a diagnosis using a neural network according to an embodiment of the present invention.
FIG. 12 is a view showing a biometric image diagnosed through a disease diagnosis system using a neural network according to an embodiment of the present invention.

### BEST MODE FOR CARRYING OUT THE INVENTION

Since the present invention may be diversely converted and have various embodiments, specific embodiments will be shown in the drawings and described in detail in the detailed description. However, it should be understood that this is not intended to limit the present invention to the specific embodiments, but to comprise all conversions, equivalents and substitutions included in the scope of the present invention. In describing the present invention, if it is determined that the detailed description on the related known art may obscure the gist of the present invention, the detailed description will be omitted.

The terms such as "first" and "second" can be used in describing various constitutional components, but the above constitutional components should not be restricted by the above terms. The above terms are used only to distinguish one constitutional component from the other.

The terms used herein are used only to describe particular embodiments and are not intended to limit the present invention. A singular expression includes a plurality of expressions, unless the context clearly indicates otherwise.

In this specification, it should be further understood that the terms "include" and "have" specify the presence of stated features, numerals, steps, operations, constitutional components, parts, or a combination thereof, but do not preclude the presence or addition of one or more other features, numerals, steps, operations, constitutional components, parts, or a combination thereof.

In the specification, when any one of constitutional components "transmits" a data to another constitutional component, it means that the constitutional component may directly transmits the data to another constitutional component or may transmit the data to another constitutional component through at least one of the other constitutional components. On the contrary, when any one of the constitutional components directly transmits a data to another constitutional component, it means that the data is transmitted to another constitutional component without passing through the other constitutional components.

Hereinafter, the present invention is described in detail focusing on the embodiments of the present invention with reference to the attached drawings. Like reference symbols presented in each drawing denote like members.

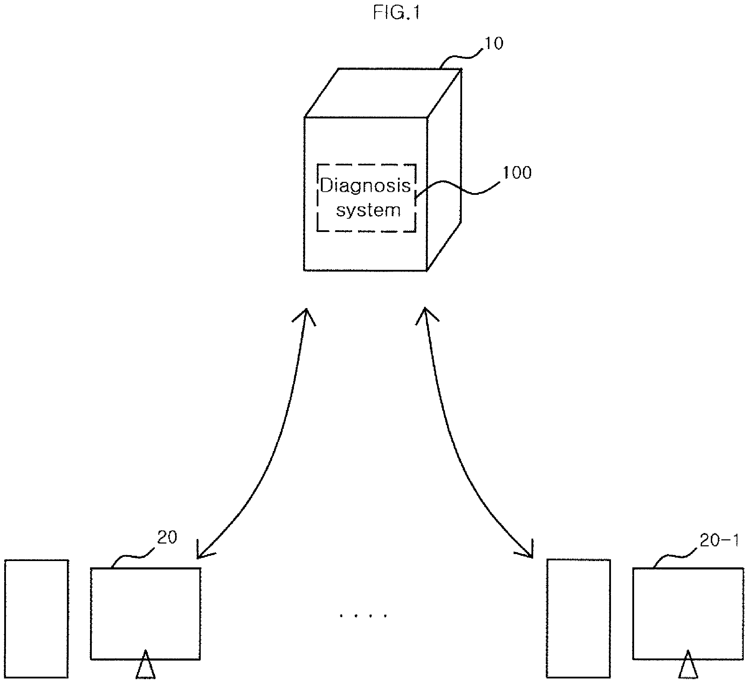

FIG. 1 is a view showing the schematic system configuration of a disease diagnosis system using a neural network according to the present invention.

Referring to FIG. 1, a disease diagnosis system using a neural network according to the present invention (hereinafter, referred to as a diagnosis system 100) may be installed in a predetermined server 10 and implement the present invention. The server 10 means a data processing device having computing capability for implementing the present invention, and those skilled in the art may easily infer that any device which can performed a specific service, such as a personal computer, a portable terminal or the like, as well as a data processing device that can be accessed by a client through a network, may be generally defined as a server.

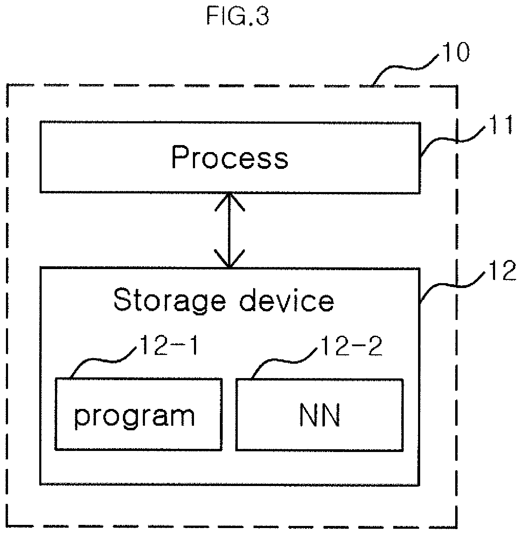

The server 10 may include a processor 11 and a storage device 12 as shown in FIG. 3. The processor 11 may means a computing device which can drive a program 12-1 for implementing the present invention, and the processor 11 may perform diagnosis using the program 12-1 and a neural network 12-2 defined by the present invention.

The storage device 12 may mean a data storage means which can store the program 12-1 and the neural network 12-2 and may be implemented as a plurality of storage means according to implementation examples. In addition, the storage device 10 may be a meaning including a temporary storage device, a memory or the like which can be included in the processor 11, as well as a main memory device included in the server 10.

Although the diagnosis system 100 is shown to be implemented as a physical device in FIG. 1 or 3, those skilled in the art may easily infer that a plurality of physical devices may be organically combined as needed to implement the diagnosis system 100 according to the present invention.

In this specification, when the diagnosis system 100 performs diagnosis, it may means a series of processes which receive a biometric image showing a biological tissue and output an output data defined in this specification. The output data may mean information outputted from an output layer of a neural network used by the diagnosis system 100, and the output data may include state information showing a state of a specific disease of a biological tissue corresponding to a tile included in the biometric image. The state information may be information outputted from a state channel included in the output layer.

For example, the state information may be probability information on whether a specific disease (e.g., a specific type of cancer) is manifested in a tissue corresponding to the tile. Or, as described below, the state information may be information indicating a degree of progression of a specific disease (or a probability of being corresponding to the degree of progression), in addition to simply indicating whether the specific disease is manifested. For example, when the present invention is used for diagnosis of prostate cancer as described below, a Gleason pattern or a Gleason score, which are indexes for indicating a degree of progression of prostate cancer, may be included in the state information. For example, the Gleason pattern has a value between 2 and 5, and it shows that the larger the value is, the higher the degree of the manifested prostate cancer. Accordingly, the state information may mean a probability that a biological tissue corresponding to a tile, which is a target of diagnosis, corresponds to a specific value (e.g., 3, 4 or 5) of the Gleason pattern.

The state information may exist in plurality. For example, first state information may indicate a probability of the Gleason pattern for being 3, second state information may indicate a probability of the Gleason pattern for being 4, and third state information may indicate a probability of the Gleason pattern for being 5. All the state channels corresponding to the first state information, the second state information and the third state information may be defined in the output layer. According to implementation examples, state information indicating that the Gleason pattern has a probability of a predetermined range (e.g., 3 to 5, 4 to 5 or the like) may be defined. That is, a piece of state information may correspond to a plurality of indexes indicating a progression state of a disease.

It is apparent that the state channel included in the output layer may be determined according to how a corresponding tile is annotated for learning.

Meanwhile, according to the present invention, correlation factor channels, as well as the state channels, may be included in the output layer. This will be described below.

When the diagnosis system 100 is implemented to be included in a predetermined server 10, the diagnosis system 100 may perform communication with at least a client (e.g., 20 or 20-1) which can access the server 10. In this case, the client (e.g., 20 or 20-1) may transmit a biometric image to the diagnosis system 100, and the diagnosis system 100 may perform diagnosis according to the present invention on the transmitted biometric image. In addition, the diagnosis system 100 may transmit a diagnosis result to the client (e.g., 20 or 20-1).

The diagnosis system 100 may perform diagnosis using a neural network according to the present invention. Of course, the diagnosis system 100 may first perform a process for training the neural network to perform the diagnosis.

Accordingly, the diagnosis system 100 may be a system which receives a neural network trained according to the present invention and a program for performing diagnosis using the neural network from the outside and performs the diagnosis or a system which also performs training the neural network. In addition, the diagnosis system 100 may be implemented as a dedicated device manufactured to implement the present invention, not a general-purpose data processing device, and in this case, a means for scanning the biometric image may be further provided.

The neural network used by the diagnosis system 100 according to the present invention may include a micro-neural network and a macro-neural network.

The micro-neural network may mean a network which performs a series of processes for performing training using a specific tile and performing diagnosis on the tile using image features of the tile itself.

The macro-neural network may mean a network which performs a series of processes for performing training using the tile and a macro-tile including the tile and at least one of tiles adjacent to the tile and performing diagnosis on the tile using image features of the entire macro-tile.

Accordingly, the neural network according to the present invention may have a characteristic of performing diagnosis on a specific tile, considering not only an image of the specific tile itself, but also an image of at least one of tiles adjacent to the specific tile, to perform diagnosis on the specific tile. Through the present invention like this, there is an effect of enhancing accuracy to a very meaningful level in the diagnosis of a disease that should consider not only a biological tissue actually corresponding to a specific tile, but also the states of tissues around the biological tissue, for diagnosis of the biological tissue. In addition, when a biometric image is divided into a plurality of tiles, this may have a strong effect on the influence of a diagnosis result which can be generated according to the method of dividing the tiles or the position of a biological tissue corresponding to a divided area.

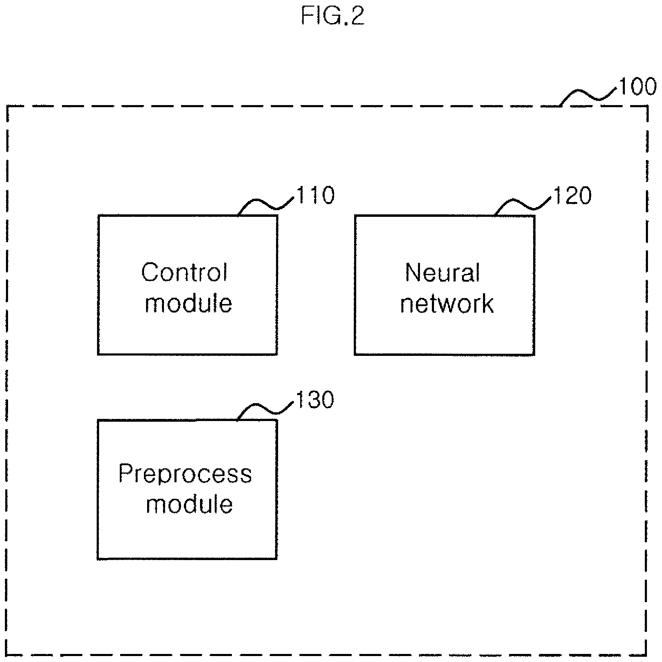

Logically, a diagnosis system 100 for implementing the present invention may have the configuration as shown in FIG. 2.

FIG. 2 is a view showing the logical configuration of a disease diagnosis system using a neural network according to an embodiment of the present invention.

Referring to FIG. 2, the diagnosis system 100 includes a control module 110 and a neural network module 120 in which a neural network is stored. In addition, the diagnosis system 100 may further include a preprocess module 130.

The diagnosis system 100 may mean a logical configuration provided with hardware resources and/or software needed for implementing the present invention and does not necessarily mean a physical configuration component or a device. That is, the diagnosis system 100 may mean a logical combination of hardware and/or software provided to implement the present invention, and if needed, the diagnosis system 100 may be installed in the devices separated from each other and separately perform its function to be implemented as a set of logical configurations for implementing the present invention. In addition, the diagnosis system 100 may mean a set of configurations separately implemented for each function or role for implementing the present invention. For example, the control module 110, the neural network module 120 and/or the preprocess module 130 may be located in different physical devices or in the same physical device. In addition, according to implementation examples, combinations of software and/or hardware configuring the control module 110, the neural network module 120 and/or the preprocess module 130 may also be located in different physical devices, and each of the modules may be implemented as the configurations located in the different physical devices are organically combined with each other.

In addition, a module in this specification may mean a functional or structural combination of hardware for performing the present invention and software for driving the hardware. For example, the module may mean a logical unit of a predetermined code and hardware resources for performing the predetermined code, and those skilled in the art may easily infer that the module does not necessarily mean physically connected codes or a kind of hardware.

The control module 110 may control other configurations (e.g., the neural network module 120 and/or the preprocess module 130) included in the diagnosis system 100 to implement the present invention.

In addition, the control module 110 may perform diagnosis according to the present invention by using the neural network stored in the neural network module 120. Performing diagnosis may mean outputting a channel value of at least one channel defined in the output layer as described above. Each channel value may indicate the probability that a tile, which is a target of diagnosis, corresponds to the information defined by the corresponding channel.

The neural network module 120 may store a neural network. The neural network may mean a set of information expressing a series of design considerations defining the neural network. In this specification, the neural network may be a convolution neural network.

As is well-known, the convolution neural network may include an input layer, a plurality of hidden layers, and an output layer. Each of the plurality of hidden layers may include a convolution layer and a pooling layer (or a sub-sampling layer).

The convolution neural network may be defined by a function defining each of the layers, a filter, a stride, a weight factor and so on. In addition, the output layer may be defined as a fully connected feed-forward layer.

Design considerations of each layer configuring the convolution neural network are well-known. For example, known functions may be used for the number of layers to be included in a plurality of layers, a convolution function for defining the plurality of layers, a pooling function, and an activation function, or functions separately defined to implement the present invention may be used.

A discrete convolution sum or the like is an example of the convolution function. Max pooling, average pooling or the like may be used as an example of the pooling function. Examples of the activation function may be a sigmoid function, a tangent hyperbolic (tanh) function, a rectified linear unit (ReLU) and the like.

When design considerations of the convolution neural network are defined, the convolution neural network, for which the design considerations are defined, may be stored in the storage unit. In addition, if the convolution neural network is trained, a weight factor corresponding to each layer may be specified.

Training of the convolution neural network may mean a process of determining weight factors of the layers. In addition, if the convolution neural network is trained, the trained convolution neural network may receive an input data through the input layer and output an output data through the output layer defined in advance.

The neural network according to an embodiment of the present invention may be defined by selecting any one or a plurality among widely-known design considerations as described above, or independent design considerations may be defined for the neural network.

The control module 110 may input an input data into the neural network stored in the neural network module 120, i.e., the trained neural network. In addition, the control module 110 may output an output data by performing computations defined by the neural network.

The preprocess module 130 may perform preprocess on a biometric image as needed before performing diagnosis using the neural network. For example, preprocess of the biometric image may include a process of tiling the biometric image into tiles of a predefined size, and those skilled in the art may easily infer that proper image processing may be performed as needed in a method appropriate to the neural network.

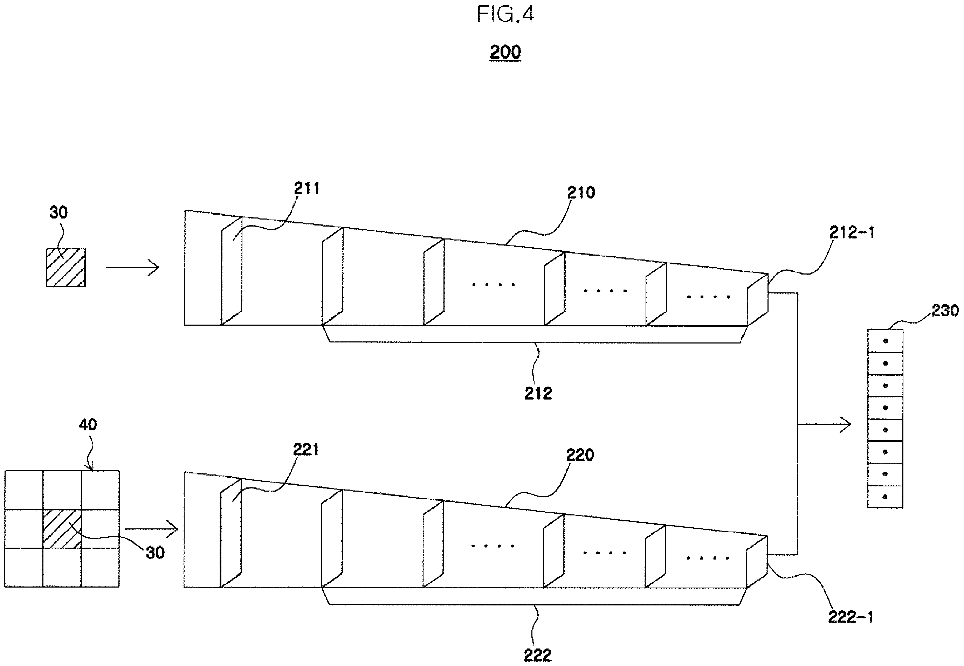

Meanwhile, a neural network according to the present invention has a characteristic of including a micro-neural network and a macro-neural network as described above. The example like this will be described in detail with reference to FIG. 4.

FIG. 4 is a view showing the configuration of a neural network according to an embodiment of the present invention.

Referring to FIG. 4, a neural network 200 according to the present invention includes a micro-neural network and a macro-neural network.

The micro-neural network includes a plurality of layers 210 and an output layer 230. An input layer 211 and a plurality of hidden layers 212 are included in the plurality of layers 210.

The macro-neural network includes a plurality of layers 220 and the output layer 230. An input layer 221 and a plurality of hidden layers 222 are included in the plurality of layers 220.

The micro-neural network is defined to receive a specific tile 30 and output a diagnosis result of the specific tile, i.e., output data defined in the output layer 230.

In addition, the macro-neural network is defined to receive a macro-tile 40 including the specific tile 30 and at least one of tiles adjacent to the specific tile 30 and output a diagnosis result of the specific tile.

That is, the neural network 200 according to the present invention my output a diagnosis result considering image features of the tiles adjacent to the specific tile 30, as well as image features of the specific tile 30, to output a diagnosis result of the specific tile 30.

Although FIG. 4 shows an example of using tiles surrounding a tile as the macro-tile 40, it is apparent that diverse embodiments are possible.

The output layer 230 may receive output data of a first right-before layer 212-1, which is a layer included in the micro-neural network right before the output layer 230, and output data of a second right-before layer 222-1, which is a layer right included in the macro-neural network before the output layer 230, and output an output data defined in the output layer 230. The first right-before layer 212-1, the second right-before layer 222-1, and the output layer 230 may be fully connected.

Any one of various functions which outputs an input data received through the input layer to the output layer 230 as an output data through the neural network 200 as a result may be used as a feed-forward function which defines the output layer 230.

As a result, the neural network 200 is trained to output an output data of the output layer 230 corresponding to annotation values of a plurality of training data, considering image features of a specific tile 30 and image features of the macro-tile 40 including the specific tile 30, to perform diagnosis on the specific tile 30.

That is, a plurality of training data is used to train the neural network 200, and the plurality of training data may include a specific tile 30 and a macro-tile 40 in pairs. In addition, the macro-tile 40 may also perform training using annotation information of the specific tile 30.

Then, the neural network 200 may be trained to output an output data corresponding to the annotation information of the specific tile 30, considering the image features of the specific tile 30 and the macro-tile 40.

In addition, when the trained neural network 200 receives a target tile, which is a target of diagnosis, and a macro-tile corresponding to the target tile as input data of the input layers of the micro neural network and the macro-neural network, the neural network 200 may output a diagnosis result of the target tile, i.e., an output data of the output layer 230.

Meanwhile, the macro-neural network may have a big stride compared with the micro-neural network. This may mean that the macro-neural network further intermittently extracts image features included in the macro-tile 40 compared with the micro-neural network. This may mean that since the macro-neural network also diagnoses the specific tile 30, it does not need to extract image features very densely and go through a process of abstracting the extracted image features like the micro-neural network. In addition, rather, it may also have an effect of limiting, to some extent, too much effect of the image features of adjacent tiles, not the specific tile 30, on the diagnosis of the specific tile 30. Accordingly, a further efficient network design can be made by setting a stride value of the macro-neural network to be larger than that of the micro-neural network. According to an embodiment, the stride value used in the micro-neural network may be 1, and a value of 2 or 3 may be used as the stride value in the macro-neural network.

In addition, a larger stride value does not need to be commonly applied to all hidden layers, and a stride value applied only to some convolution layers of early stage may be larger in the macro-neural network than in the micro-neural network.

Meanwhile, the output layer 230 may output a diagnosis result of the specific tile 30, which is a target of diagnosis, as an output data. The diagnosis result may include at least information on the state of a disease of the specific tile 30. Information on the state of a disease may simply mean information on whether a specific disease is manifested (or a probability value) in the specific tile 30.

However, information indicating a degree of progression of a disease in further detail may be included in the information on the state of a disease according to disease type. Hereinafter, although a case in which the type of a disease diagnosed by the diagnosis system 100 is prostate cancer is described as an example, those skilled in the art may easily infer that the present invention does not necessarily need to be applied only to the prostate cancer.

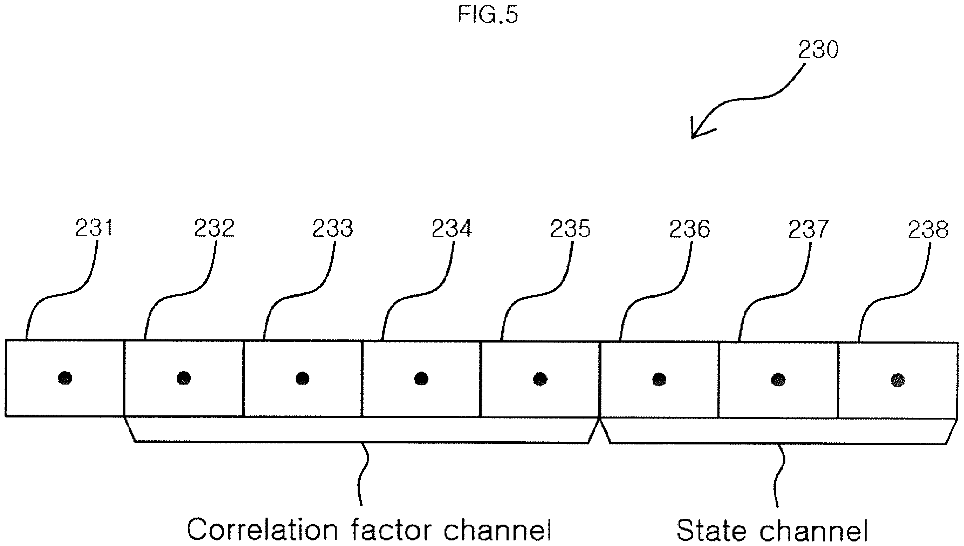

FIG. 5 is a view showing an output layer of a neural network according to an embodiment of the present invention.

Referring to FIG. 5, an output layer 230 according to an embodiment of the present invention may include at least one channel (e.g., 231 to 238).

The output layer 230 may include at least a state channel (e.g., 236, 237 or 238) indicating information on the state of a disease as described above. The state channels (e.g., 236, 237 and 238) may be information indicating a degree of progression of a disease, respectively. For example, a first state channel 236 may be information indicating a probability of having a first value (e.g., Gleason pattern of 3), which is any one among the values of Gleason pattern, i.e., an index indicating a degree of progression of prostate cancer. A second state channel 237 and a third state channel 238 may be information indicating probabilities of having a second value (e.g., Gleason pattern of 4) and a third value (e.g., Gleason pattern of 5), which are different values among the values of Gleason pattern. When an index indicating a degree of progression is defined according to the type of a disease like this, there is an effect of diagnosing a degree of progression of a corresponding disease, in addition to simply diagnosing whether a disease is manifested.

Meanwhile, according to present invention, at least one of correlation factor channels (e.g., 232, 233, 234 and 235) indicating a degree of manifestation of a correlation factor associated with a value of the state channel, as well as information on whether a disease is manifested in a tile, which is a target of diagnosis, or how much a disease has progressed, may be further included in the output layer 230.

That is, the correlation factor channels (e.g., 232, 233, 234 and 235) may be information indicating whether individual factors, which are the basis for determining whether a disease is manifested or a degree of progression of the disease, are manifested. For example, manifestation of prostate cancer or a degree of progression thereof may be determined by considering one or more individual factors, such as whether the state of a cell nucleus satisfies a predetermined condition, whether the gland cell wall is made up of a single layer, whether the gland is concentrated more than a predetermined level, and/or whether the shape of stroma is normal.

That is, a channel value of the channel state is determined by combination of individual factors, and according to the present invention, the individual factors may be defined as separate channels, and whether the individual factors are manifested is also diagnosed through training.

If the correlation factor channels (e.g., 232, 233, 234 and 235) are defines in the output layer 230 like this, the problem that the medical staff does not know, when only the state channel actually exists, how the output value of the corresponding state channel has been derived can be solved. That is, according to the present invention, there is an effect of additionally providing an output value of the state channel, e.g., whether individual factors are manifested, which is a basis for determining, by the diagnosis system 100, that a disease is manifested or the disease is progressed to some extent, i.e., providing output values of the correlation factor channels (e.g., 232, 233, 234 and 235). Therefore, there is an effect of providing further higher reliability of the diagnosis result of the diagnosis system 100. In addition, since it can be easily confirmed whether the output value of the correlation factor channel (e.g., 232, 233, 234 and 235) and the output value of the state channel match each other, there is an effect of determining, during the training, whether training of the diagnosis system 100 is normally progressed, and thus this may be effective for training itself of the neural network 200. Furthermore, it is conformed that the neural network 200 has a further correct diagnosis rate when the correlation factor channels (e.g., 232, 233, 234 and 235) are separately trained. This may means that the neural network 200 considers individual factors in determining the output value of the status channel as the correlation factor channels (e.g., 232, 233, 234 and 235) are actually included in the output layer 230.

When the diagnosis system 100 is trained for diagnosis of prostate cancer, the correlation factor channel may include a channel indicating a probability that the cell nucleus of a biological tissue corresponding to the tile satisfies a specific condition. For example, the specific condition may include conditions such as a case of a relatively large nucleus, a case of a dark color, a case of a clearly visible nucleolus, a degree close to the original shape of a nucleus, and the like, and the more these conditions are satisfied, the higher the probability that prostate cancer has been developed or a degree of progression thereof is severe.

In addition, the correlation factor channels (e.g., 232, 233, 234 and 235) may include a channel indicating a probability that a biological tissue corresponding to the tile is classified as a single cell layer, i.e., classified as a case of clearly showing a gland cell wall as a single layer. When the characteristic of a single cell layer is manifested, it is highly probable that prostate cancer has been developed or a degree of progression thereof is severe.

In addition, the correlation factor channels (e.g., 232, 233, 234 and 235) may include a channel indicating a probability that a biological tissue corresponding to the tile is classified as a high density gland. For example, according to whether more than a predetermined number of glands are concentrated within a predetermined range, it may be determined that the probability of developing prostate cancer is high or a degree of progression thereof is severe.

In addition, the correlation factor channels (e.g., 232, 233, 234 and 235) may include a channel indicating a probability that a biological tissue corresponding to the tile is classified as normal stroma. That is, the probability of prostate cancer or the degree of progression may be low when the biological tissue has a shape of normal stroma.

In order for the trained neural network 200 to output each of the channel values defined in the output layer 230 as an output data, annotation values of the channels corresponding to the output layer 230 should be annotated to the training data, i.e., the tile and the macro-tile, and to this end, professional manpower capable of annotating the channel values may be required.

In an embodiment of the present invention, a predetermined channel (e.g., 231) among the channels included in the output layer 230 may not mean any meaningful information. That is, a corresponding channel may be a channel that is not used for learning and diagnosing. This is since that the output layer 230 may be designed to include at least one unused channel if it is advantageous for the output layer 230 to have as many channels as a multiple of two when a network is designed.

As a result, to diagnose any one tile, which is a target of diagnosis, the neural network 200 used by the diagnosis system 100 according to the present invention has a characteristic of performing diagnosis with further higher accuracy by considering an image of a macro-tile including the tile, as well as the tile.

In addition, since the neural network 200 includes at least a correlation factor channel, not simply outputting a state channel as an output data, reliability of a diagnosis result can be secured, and it is effective for learning of the neural network 200, and as the correlation factor channels are included in the output layer, there is an effect of enhancing the accuracy of the diagnosis result itself of the state channel.

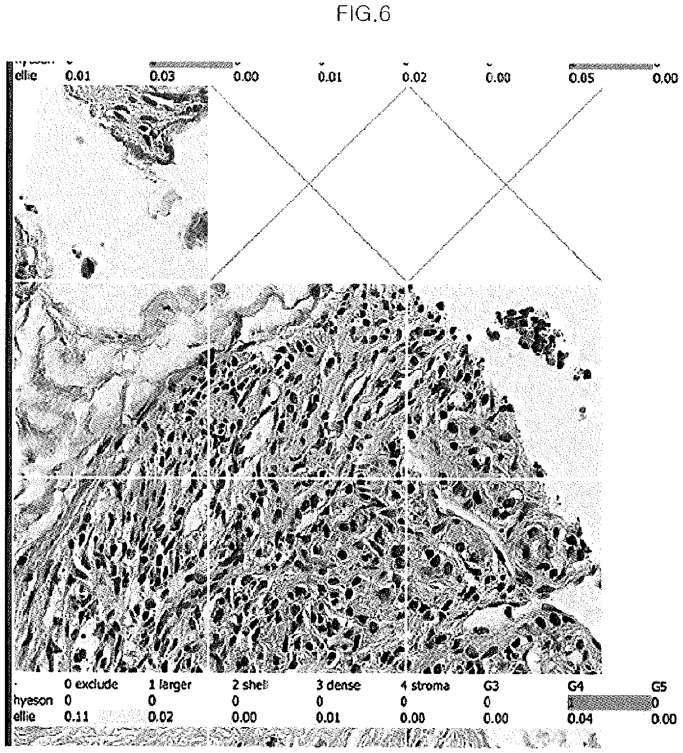

FIG. 6 is a view showing an example of annotation information for training a neural network according to an embodiment of the present invention.

Referring to FIG. 6, annotation information of a macro-tile as shown in FIG. 6 may be needed to train the neural network 200 according to the present invention. In FIG. 6, values of the channels defined in the output layer 230 are annotated for the tile existing at the center of the macro-tile. For example, eight channel values are annotated as shown in the lower portion of FIG. 6, for example, it is shown that a first channel is an unused channel, a second channel is a correlation factor channel for a cell nucleus as described above, a third channel is a correlation factor channel related to a single cell layer, a fourth channel is a correlation factor channel for a high density gland, a fifth channel is a correlation factor channel for whether stroma is a normal, a sixth channel is a state channel in which the Gleason pattern has a value of 3, a seventh channel is a state channel in which the Gleason pattern has a value of 4, and an eighth channel is a state channel in which the Gleason pattern has a value of 5. In addition, it may mean that an annotator has annotated that the Gleason pattern has a state of 4 for the tile located at the center of the macro-tile. Of course, although not shown in FIG. 6, annotation may be further performed on a correlation factor channel, which is the basis for determining that the Gleason pattern is 4.

The neural network 200 may be trained as the annotated information, the annotated tile and the macro-tile including the tile are used as training data.

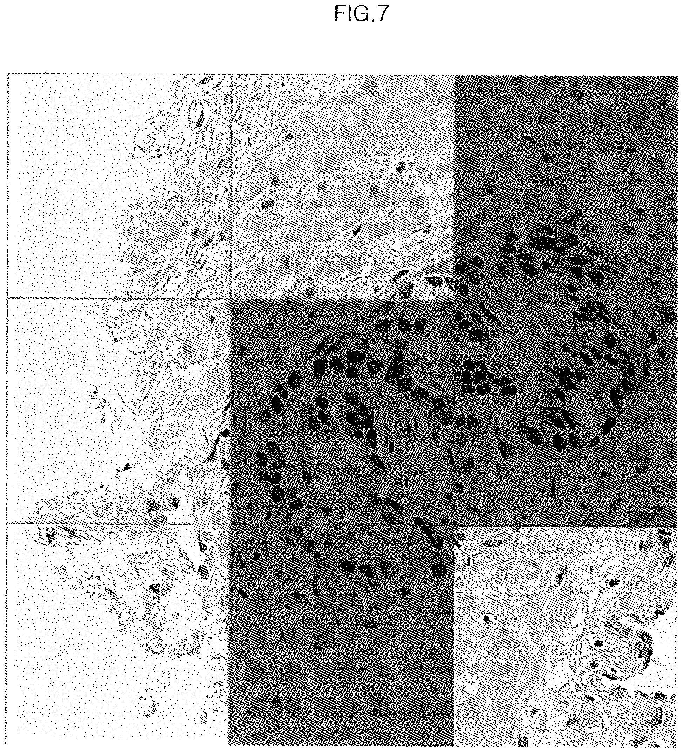

FIGS. 7 to 10 are views showing correlation factors in a neural network according to an embodiment of the present invention.

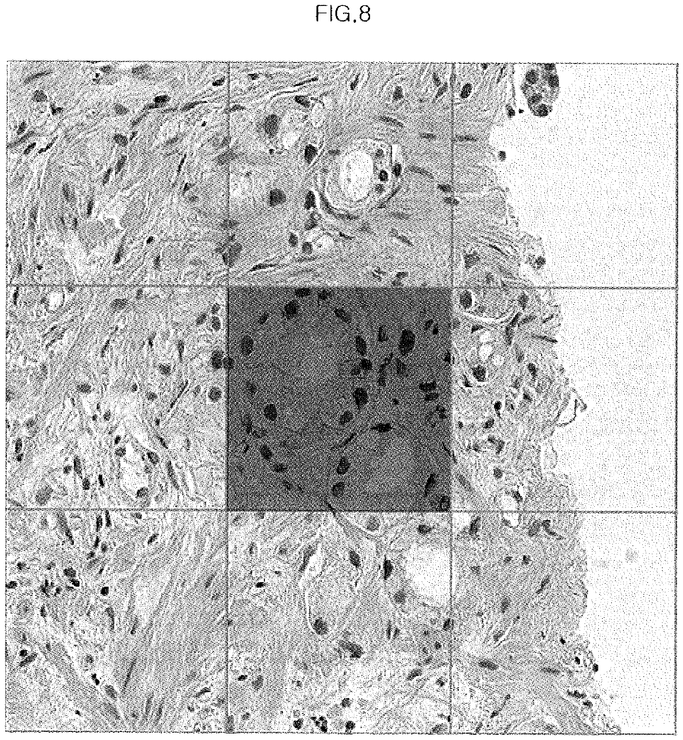

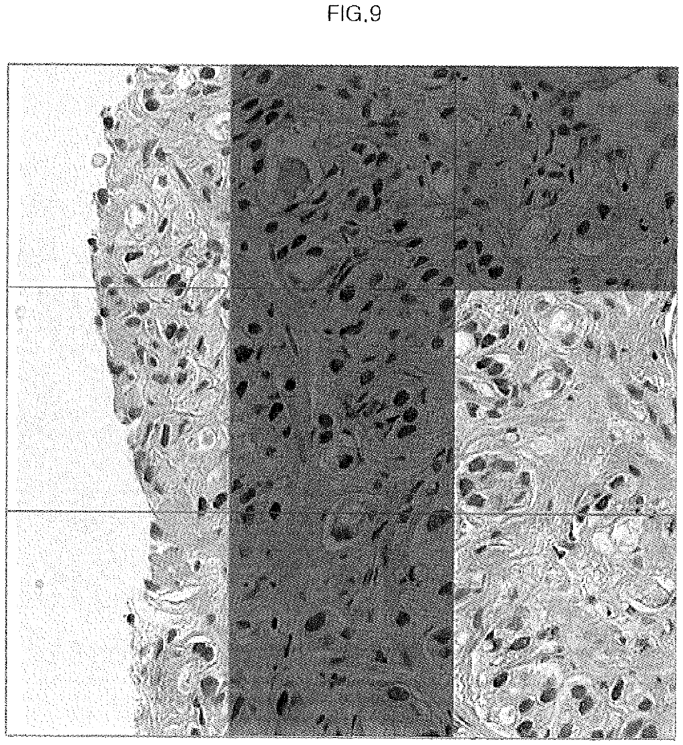

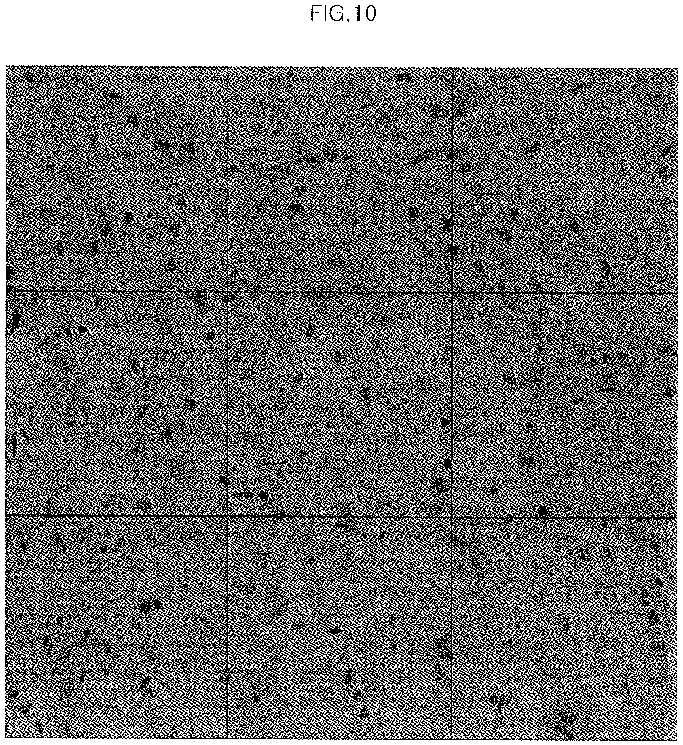

FIGS. 7 to 10 show examples of tiles in which a corresponding value is annotated (or diagnosed) to a correlation factor channel as described above. FIG. 7 shows inverted views of tiles satisfying conditions on the cell nucleus among the tiles, FIG. 8 shows inverted views of tiles corresponding to a single cell layer, FIG. 9 shows inverted views of tiles corresponding to a high density gland, and FIG. 10 shows inverted views of tiles corresponding to normal stroma.

Accordingly, according to the diagnose system 100 according to the present invention, there is an effect of extracting tiles in which a specific disease is manifested or tiles corresponding to a specific state of progression from a biometric image in which a plurality of tiles exists and an effect of separately extracting tiles in which specific individual factors are manifested.

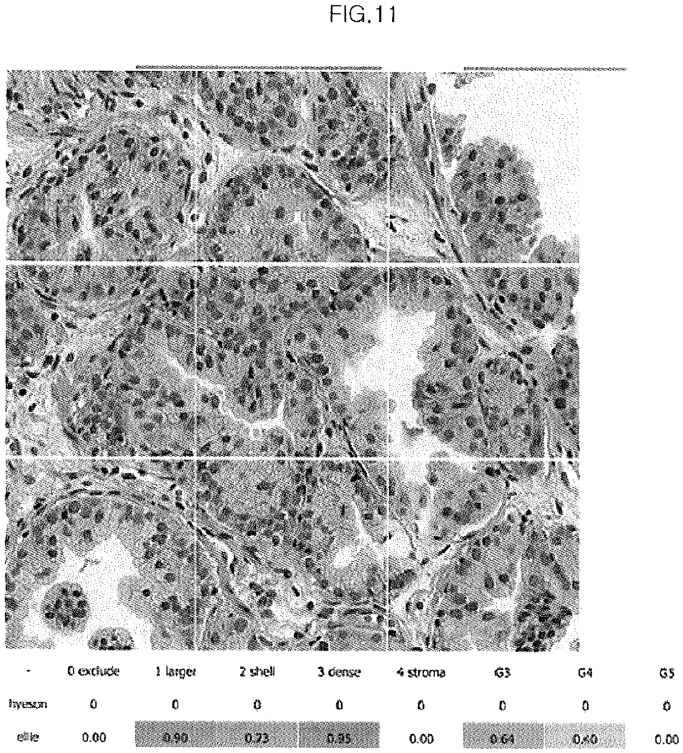

FIG. 11 is a view showing a result of a diagnosis using a neural network according to an embodiment of the present invention.

Referring to FIG. 11, a diagnosis system 100 using a neural network 200 according to an embodiment of the present invention may output, as shown in the lower portion of FIG. 11, a diagnosis result of the tile at the center among the tiles shown in FIG. 11.

For example, the diagnosis system 100 has determined that the value of a first correlation factor channel is 0.90, the value of a second correlation factor channel is 0.73, the value of a third correlation factor channel is 0.95, the value of a fourth correlation factor channel is 0, the value of a first state channel is 0.64, the value of a second state channel is 0.4, and the value of a third state channel is 0, for the tile at the center among the tiles shown in FIG. 11 using the trained neural network 200.

It is known that the tile at the center is highly probable that the Gleason pattern is 3, the cell nucleus satisfies a specific condition, the tile corresponds to a single cell layer, and the tile corresponds to a high density gland.

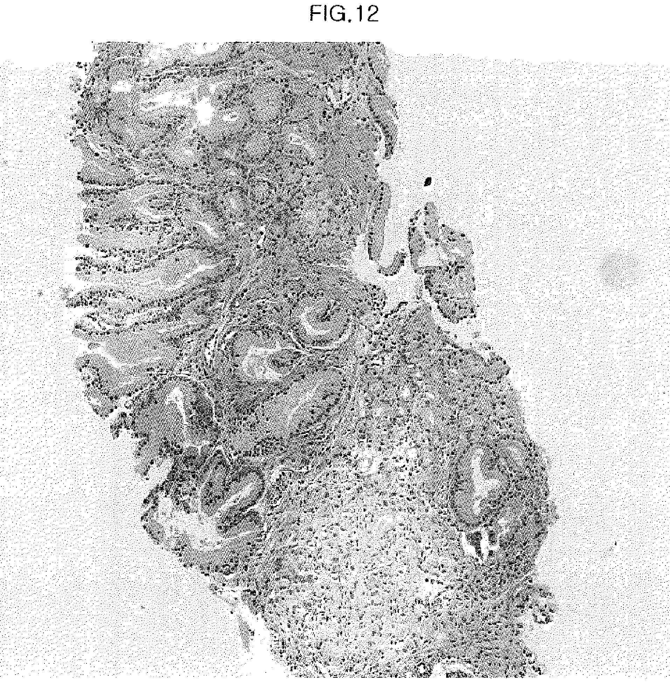

FIG. 12 is a view showing a biometric image diagnosed through a disease diagnosis system using a neural network according to an embodiment of the present invention.

FIG. 12 shows an example of a result, in which the parts where prostate cancer has developed in a biometric image are shown differently according to a degree of progression through the diagnosis system 100 according to the present invention. FIG. 12 is an example showing that the part expressed in green color is a biological tissue corresponding to Gleason pattern of 3, the part expressed in violet color is a biological tissue corresponding to Gleason pattern of 4, and the part expressed in red color is a biological tissue corresponding to Gleason pattern of 5.

Accordingly, the diagnosis system 100 according to the present invention has an effect of automatically and effectively diagnosing a disease, which has taken a long time for the existing trained pathology workers, within a short time. In addition, as it is defined to include a state channel in a plurality of output layers according to a degree of progression of a disease, there is an effect of discriminatively displaying a diagnosis result as shown in FIG. 12 according to the difference of the degree of progression of a disease in a biological tissue.

In addition, it is confirmed that accuracy of diagnosis has been enhanced further higher compared with a case of simply training only a single tile as described above and configuring a neural network performing the diagnosis using the same, as a result of diagnosing a single tile considering both the single tile and a macro-tile including the single tile.

In addition, although an example of applying the present invention to prostate cancer has been mainly described in this specification, those skilled in the art may easily infer that a correct diagnosis can be made if the present invention is also applied to other diseases which need to perform a diagnosis on a specific tissue considering the states of tissues around the corresponding tissue, as well as the state of the specific tissue.

The method of diagnosing a disease using a neural network according to an embodiment of the present invention can be implemented as a computer-readable code in a computer-readable recording medium. The computer-readable recording medium includes all kinds of recording devices for storing data that can be read by a computer system. Examples of the computer-readable recording medium are ROM, RAM, CD-ROM, a magnetic tape, a hard disk, a floppy disk, an optical data storage device and the like. In addition, the computer-readable recording medium may be distributed in computer systems connected through a network, and a code that can be read by a computer in a distributed manner can be stored and executed therein. In addition, functional programs, codes and code segments for implementing the present invention can be easily inferred by programmers in the art.

While the present invention has been described with reference to the embodiments shown in the drawings, this is illustrative purposes only, and it will be understood by those having ordinary knowledge in the art that various modifications and other equivalent embodiments can be made. Accordingly, the true technical protection range of the present invention should be defined by the attached claims.

### INDUSTRIAL APPLICABILITY

The present invention can be applied to a disease diagnosis system using a neural network and a method thereof.

## Claims

1. A disease diagnosis system (100) implemented in a system including a processor (11) and a storage device (12) for storing a neural network (200) and using a biometric image and the neural network (200), wherein the disease is a type of cancer, the disease diagnosis system (100) comprising:
a micro-neural network for receiving a predetermined tile (30) included in the biometric image through an input layer (211) and including a plurality of first layers (212) and an output layer (230), wherein a stride value is set to the micro-neural network; and
a macro-neural network for receiving a macro-tile (40) including the tile (30) and at least one of tiles adjacent to the tile (30) through an input layer (221) and including a plurality of second layers (222) and the output layer (230), wherein a stride value is set to the macro-neural network;
wherein the stride value of the macro-neural network is larger than the stride value of the micro-neural network;
wherein the output layer (230) includes at least a state channel (236, 237, 238) indicating a probability of a biological tissue corresponding to the tile (30) to have a Gleason pattern value of a predetermined range; and
wherein the output layer (230) is determined on the basis of output data of a first right-before layer (212-1) included in the first layers (212) and located right before the output layer (230) and a second right-before layer (222-1) included in the second layers (222) and located right before the output layer (230).

2. The system according to claim 1, wherein the stride value used in the micro-neural network is set to be 1, and the stride value used in the macro-neural network is set to be 2 or 3.

3. The system according to claim 1 or 2, wherein the output layer (230) includes at least a correlation factor channel (231, 232, 233, 234, 235) indicating a degree of manifestation of a correlation factor associated with a value of the state channel (236, 237, 238) .

4. The system according to any one of claims 1 to 3, wherein the disease is prostate cancer.

5. The system according to claim 3 or 4, wherein the correlation factor channel (231, 232, 233, 234, 235) includes at least a channel among a channel indicating a probability that a cell nucleus of a biological tissue corresponding to the tile (30) satisfies a specific condition; a channel indicating a probability that a biological tissue corresponding to the tile (30) is classified as a single cell layer; a channel indicating a probability that a biological tissue corresponding to the tile (30) is classified as a high density gland; and a channel indicating a probability that a biological tissue corresponding to the tile (30) is classified as normal stroma.

6. A computer-implemented method of diagnosing a disease using a neural network (200), performed by the disease diagnosis system (100) according to any one of claims 1 to 5 implemented in a system including a processor (11) and a storage device (12) and using a biometric image and the neural network (200), wherein the disease is a type of cancer, the method comprising the steps of:
storing the micro-neural network for receiving a predetermined tile (30) included in the biometric image through an input layer (211) and including a plurality of first layers (212) and an output layer (230), wherein a stride value is set to the micro-neural network; and
storing the macro-neural network for receiving a macro-tile (40) including the tile (30) and at least one of tiles adjacent to the tile (30) through an input layer (221) and including a plurality of second layers (222) and the output layer (230), wherein a stride value is set to the macro-neural network; and
training the micro-neural network and the macro-neural network using annotation information annotated to the tile (30) to correspond to the output layer (30);
wherein the stride value of the macro-neural network is larger than the stride value of the micro-neural network;
wherein the output layer (30) includes at least the state channel (236, 237, 238) indicating a probability of a biological tissue corresponding to the tile (30) to have a Gleason pattern value of a predetermined range;
wherein the output layer (230) is determined on the basis of output data of a first right-before layer (212-1) included in the first layers (212) and located right before the output layer (230) and a second right-before layer (222-1) included in the second layers (222) and located right before the output layer (230).

7. The computer-implemented method according to claim 6, further comprising the steps of:
receiving a target tile included in a diagnosis target biometric image, by the neural network (200) including the trained micro-neural network and the trained macro-neural network; and
outputting an output data corresponding to the output layer (230) through the neural network (200).

8. The computer-implemented method according to claim 6 or 7, wherein the stride value used in the micro-neural network is set to be 1, and the stride value used in the macro-neural network is set to be 2 or 3.

9. The computer-implemented method according to any one of claims 6 to 8, wherein the output layer (230) includes at least a correlation factor channel (231, 232, 233, 234, 235) indicating a degree of manifestation of a correlation factor associated with a value of the state channel (236, 237, 238).

10. The computer-implemented method according to any one of claims 6 to 9, wherein the disease is prostate cancer.

11. A computer program installed on a data processing device and recorded in a medium to perform the method disclosed in any one of claims 6 to 10.

## Patentansprüche

1. Ein Krankheitsdiagnosesystem (100), das in einem System implementiert ist, das einen Prozessor (11) und eine Speichervorrichtung (12) zum Speichern eines neuronalen Netzwerks (200) und zur Verwendung eines biometrischen Bildes und des neuronalen Netzwerks (200) enthält, wobei die Krankheit ein Krebstyp ist, wobei das Krankheitsdiagnosesystem (100) umfasst:
ein mikroneuronales Netzwerk zum Empfangen einer vorbestimmten Kachel (30), die in dem biometrischen Bild enthalten ist, durch eine Eingabeschicht (211) und mit einer Vielzahl von ersten Schichten (212) und einer Ausgabeschicht (230), wobei ein Stride-Wert für das mikroneuronale Netzwerk festgelegt wird; und
ein makroneuronales Netzwerk zum Empfangen einer Makrokachel (40), welche die Kachel (30) und mindestens eine der an die Kachel (30) angrenzenden Kacheln enthält, durch eine Eingabeschicht (221), die eine Vielzahl von zweiten Schichten (222) und die Ausgabeschicht (230) enthält, wobei dem makroneuronalen Netzwerk ein Stride-Wert zugewiesen wird;
wobei der Stride-Wert des makroneuronalen Netzwerkes größer ist als der Stride-Wert des mikroneuronalen Netzwerkes;
wobei die Ausgabeschicht (230) mindestens einen Zustandskanal (236, 237, 238) enthält, der eine Wahrscheinlichkeit anzeigt, dass ein biologisches Gewebe, das der Kachel (30) entspricht, einen Gleason-Musterwert in einem vorbestimmten Bereich aufweist; und
wobei die Ausgabeschicht (230) auf der Grundlage von Ausgabedaten einer ersten Unmittelbar-vor-Schicht (212-1), die in den ersten Schichten (212) enthalten und unmittelbar vor der Ausgabeschicht (230) angeordnet ist, und einer zweiten Unmittelbar-vor-Schicht (222-1), die in den zweiten Schichten (222) enthalten und unmittelbar vor der Ausgabeschicht (230) angeordnet ist, bestimmt wird.

2. System nach Anspruch 1, wobei der im mikroneuronalen Netzwerk verwendete Stride-Wert auf 1 und der im makroneuronalen Netzwerk verwendete Stride-Wert auf 2 oder 3 eingestellt ist.

3. System nach Anspruch 1 oder 2, wobei die Ausgabeschicht (230) mindestens einen Korrelationsfaktorkanal (231, 232, 233, 234, 235) enthält, der einen Manifestationsgrad eines Korrelationsfaktors anzeigt, der mit einem Wert des Zustandskanals (236, 237, 238) verbunden ist.

4. System nach einem der Ansprüche 1 bis 3, wobei die Krankheit Prostatakrebs ist.

5. System nach Anspruch 3 oder 4, wobei der Korrelationsfaktorkanal (231, 232, 233, 234, 235) mindestens einen Kanal unter Folgenden umfasst, einen Kanal, der eine Wahrscheinlichkeit anzeigt, dass ein Zellkern eines biologischen Gewebes, das der Kachel (30) entspricht, eine spezifische Bedingung erfüllt; einen Kanal, der eine Wahrscheinlichkeit anzeigt, dass ein biologisches Gewebe, das der Kachel (30) entspricht, als eine Einzelzellschicht klassifiziert ist; einen Kanal, der eine Wahrscheinlichkeit anzeigt, dass ein biologisches Gewebe, das der Kachel (30) entspricht, als eine Drüse mit hoher Dichte klassifiziert ist; und einem Kanal, der eine Wahrscheinlichkeit anzeigt, dass ein biologisches Gewebe, das der Kachel (30) entspricht, als normales Stroma klassifiziert ist.

6. Computerimplementiertes Verfahren zur Diagnose einer Krankheit unter Verwendung eines neuronalen Netzwerkes (200), das von dem Krankheitsdiagnosesystem (100) nach einem der Ansprüche 1 bis 5 durchgeführt wird, das in einem System implementiert ist, das einen Prozessor (11) und eine Speichervorrichtung (12) enthält, und das ein biometrisches Bild und das neuronale Netzwerk (200) verwendet, wobei die Krankheit eine Art von Krebs ist, wobei das Verfahren die folgenden Schritte umfasst:
Speichern des mikroneuronalen Netzwerkes zum Empfangen einer vorbestimmten Kachel (30), die in dem biometrischen Bild enthalten ist, durch eine Eingabeschicht (211), die eine Vielzahl von ersten Schichten (212) und eine Ausgabeschicht (230) umfasst, wobei dem mikroneuronalen Netzwerk ein Stride-Wert zugewiesen wird; und
Speichern des makroneuronalen Netzwerkes zum Empfangen einer Makrokachel (40), welche die Kachel (30) und mindestens eine der an die Kachel (30) angrenzenden Kacheln enthält, durch eine Eingabeschicht (221), die eine Vielzahl von zweiten Schichten (222) und die Ausgabeschicht (230) enthält, wobei dem makroneuronalen Netzwerk ein Stride Wert zugewiesen wird; und
Trainieren des mikroneuronalen Netzwerkes und des makroneuronalen Netzwerkes unter Verwendung von Anmerkungsinformationen, die der Kachel (30) zugeordnet sind, um der Ausgabeschicht (30) zu entsprechen;
wobei der Stride-Wert des makroneuronalen Netzwerkes größer ist als der Stride-Wert des mikroneuronalen Netzwerkes;
wobei die Ausgabeschicht (30) mindestens den Zustandskanal (236, 237, 238) enthält, der eine Wahrscheinlichkeit anzeigt, dass ein biologisches Gewebe, das der Kachel (30) entspricht, einen Gleason-Musterwert in einem vorbestimmten Bereich aufweist;
wobei die Ausgabeschicht (230) auf der Grundlage von Ausgabedaten einer ersten Unmittelbar-vor-Schicht (212-1), die in den ersten Schichten (212) enthalten und unmittelbar vor der Ausgabeschicht (230) angeordnet ist, und einer zweiten Unmittelbar-vor-Schicht (222-1), die in den zweiten Schichten (222) enthalten und unmittelbar vor der Ausgabeschicht (230) angeordnet ist, bestimmt wird.

7. Computerimplementiertes Verfahren nach Anspruch 6, das außerdem die folgenden Schritte umfasst:
Empfangen einer Zielkachel, die in einem biometrischen Diagnosezielbild enthalten ist, durch das neuronale Netzwerk (200), das das trainierte mikroneuronale Netzwerk und das trainierte makroneuronale Netzwerk enthält; und
Ausgeben von Ausgangsdaten, die der Ausgangsschicht (230) entsprechen, durch das neuronale Netzwerk (200).

8. Computerimplementiertes Verfahren nach Anspruch 6 oder 7, wobei der im mikroneuronalen Netzwerk verwendete Stride-Wert auf 1 und der im makroneuronalen Netzwerk verwendete Stride-Wert auf 2 oder 3 gesetzt wird.

9. Computerimplementiertes Verfahren nach einem der Ansprüche 6 bis 8, wobei die Ausgabeschicht (230) mindestens einen Korrelationsfaktorkanal (231, 232, 233, 234, 235) enthält, der einen Manifestationsgrad eines Korrelationsfaktors anzeigt, der mit einem Wert des Zustandskanals (236, 237, 238) verbunden ist.

10. Computerimplementiertes Verfahren nach einem der Ansprüche 6 bis 9, wobei die Krankheit Prostatakrebs ist.

11. Computerprogramm, das auf einer Datenverarbeitungsvorrichtung installiert und auf einem Medium aufgezeichnet ist, um das in einem der Ansprüche 6 bis 10 offenbarte Verfahren durchzuführen.

## Revendications

1. Un système de diagnostic de maladie (100) mis en oeuvre dans un système comprenant un processeur (11) et un dispositif de stockage (12) pour stocker un réseau neuronal (200) et utiliser une image biométrique et le réseau neuronal (200), dans lequel la maladie est un type de cancer, le système de diagnostic de maladie (100) comprenant :
un réseau micro-neuronal pour recevoir une tuile prédéterminée (30) incluse dans l'image biométrique à travers une couche d'entrée (211) et comprenant une pluralité de premières couches (212) et une couche de sortie (230), dans lequel une valeur de stride est définie pour le réseau micro-neural; et
un réseau macro-neuronal pour la réception d'une macro-tuile (40) comprenant la tuile (30) et au moins une des tuiles adjacentes à la tuile (30) à travers une couche d'entrée (221) et comprenant une pluralité de secondes couches (222) et la couche de sortie (230), dans lequel une valeur de stride est définie pour le réseau macro-neural;
dans lequel la valeur de stride du réseau macro-neuronal est supérieure à la valeur de stride du réseau micro-neuronal;
dans laquelle la couche de sortie (230) comprend au moins un canal d'état (236, 237, 238) indiquant la probabilité qu'un tissu biologique correspondant à la tuile (30) ait une valeur de Gleason dans une plage prédéterminée; et
la couche de sortie (230) est déterminée sur la base des données de sortie d'une première couche juste avant (212-1) incluse dans les premières couches (212) et située juste avant la couche de sortie (230) et d'une deuxième couche juste avant (222-1) incluse dans les deuxièmes couches (222) et située juste avant la couche de sortie (230) .

2. Le système selon la revendication 1, dans lequel la valeur de stride utilisée dans le réseau micro-neuronal est fixée à 1, et la valeur de stride utilisée dans le réseau macro-neuronal est fixée à 2 ou 3.

3. Le système selon la revendication 1 ou 2, dans lequel la couche de sortie (230) comprend au moins un canal de facteur de corrélation (231, 232, 233, 234, 235) indiquant un degré de manifestation d'un facteur de corrélation associé à une valeur du canal d'état (236, 237, 238) .

4. Le système selon l'une des revendications 1 à 3, dans lequel la maladie est le cancer de la prostate.

5. Le système selon la revendication 3 ou 4, dans lequel le canal de facteur de corrélation (231, 232, 233, 234, 235) comprend au moins un canal parmi un canal indiquant une probabilité qu'un noyau cellulaire d'un tissu biologique correspondant à la tuile (30) satisfasse à une condition spécifique; un canal indiquant la probabilité qu'un tissu biologique correspondant à la tuile (30) soit classé comme une couche monocellulaire; un canal indiquant la probabilité qu'un tissu biologique correspondant à la tuile (30) soit classé comme une glande à haute densité; et un canal indiquant la probabilité qu'un tissu biologique correspondant à la tuile (30) soit classé comme un stroma normal.

6. Méthode informatisée de diagnostic d'une maladie à l'aide d'un réseau neuronal (200), exécutée par le système de diagnostic de maladie (100) selon l'une quelconque des revendications 1 à 5, mis en oeuvre dans un système comprenant un processeur (11) et un dispositif de stockage (12) et utilisant une image biométrique et le réseau neuronal (200), dans laquelle la maladie est un type de cancer, la méthode comprenant les étapes suivantes:
stocker le réseau micro-neuronal pour recevoir une tuile prédéterminée (30) incluse dans l'image biométrique par l'intermédiaire d'une couche d'entrée (211) et comprenant une pluralité de premières couches (212) et une couche de sortie (230), dans laquelle une valeur de stride est définie pour le réseau micro-neural; et
stocker le réseau macro-neuronal pour recevoir une macro-tuile (40) comprenant la tuile (30) et au moins une des tuiles adjacentes à la tuile (30) à travers une couche d'entrée (221) et comprenant une pluralité de secondes couches (222) et la couche de sortie (230), dans laquelle une valeur de foulée est définie pour le réseau macro-neuronal; et
entraîner le réseau micro-neuronal et le réseau macro-neural à l'aide des informations d'annotation attribuées à la tuile (30) pour correspondre à la couche de sortie (30);
dans lequel la valeur de stride du réseau macro-neuronal est supérieure à la valeur de stride du réseau micro-neuronal;
la couche de sortie (30) comprend au moins le canal d'état (236, 237, 238) indiquant la probabilité qu'un tissu biologique correspondant à la tuile (30) ait une valeur de Gleason dans une plage prédéterminée;
la couche de sortie (230) est déterminée sur la base des données de sortie d'une première couche juste avant (212-1) incluse dans les premières couches (212) et située juste avant la couche de sortie (230) et d'une deuxième couche juste avant (222-1) incluse dans les deuxièmes couches (222) et située juste avant la couche de sortie (230) .

7. La méthode mise en oeuvre par ordinateur selon la revendication 6, comprenant en outre les étapes suivantes:
réception d'une tuile cible incluse dans une image biométrique cible de diagnostic, par le réseau neuronal (200) comprenant le micro-réseau neuronal entraîné et le macro-réseau neuronal entraîné; et
émettre une donnée de sortie correspondant à la couche de sortie (230) par l'intermédiaire du réseau neuronal (200) .

8. La méthode mise en oeuvre par ordinateur selon la revendication 6 ou 7, dans laquelle la valeur de stride utilisée dans le réseau micro-neuronal est fixée à 1, et la valeur de stride utilisée dans le réseau macro-neuronal est fixée à 2 ou 3.

9. La méthode mise en oeuvre par ordinateur selon l'une des revendications 6 à 8, dans laquelle la couche de sortie (230) comprend au moins un canal de facteur de corrélation (231, 232, 233, 234, 235) indiquant un degré de manifestation d'un facteur de corrélation associé à une valeur du canal d'état (236, 237, 238).

10. La méthode mise en oeuvre par ordinateur selon l'une des revendications 6 à 9, dans laquelle la maladie est le cancer de la prostate.

11. Programme d'ordinateur installé sur un dispositif de traitement des données et enregistré sur un support pour exécuter la méthode décrite dans l'une des revendications 6 à 10.
